# DEMANDE DE BREVET EUROPEEN

(11) **EP 4 800 028 A1**
(43) Date de publication de la demande: **02.09.2026**
(21) Numéro de dépôt: 25305252.6
(22) Date de dépôt: 26.02.2025
(51) Int. Cl.: C07K 14/445, C07K 14/395, C07K 14/47, C12N 15/62

(54) **SYSTEME DE CYCLISATION DE PROTEINES RECOMBINANTES POUR LEUR EXPRESSION ET PURIFICATION**

(71) Demandeur: Université de Lorraine, 54000 Nancy (FR); Centre National de la Recherche Scientifique, 75016 Paris (FR); Institut National de la Santé et de la Recherche Médicale, 75013 Paris (FR)
(72) Inventeur: CHAGOT, Marie-Eve, 54000 NANCY (FR); QUINTERNET, Marc, 54230 CHALIGNY (FR)
(74) Mandataire: Novagraaf Technologies

(57) **Abrégé**

L'invention concerne un nouveau procédé de cyclisation d'une protéine d'intérêt utilisant une étiquette protéique constituée d'un peptide spécifique (NUFIP1 ou TAH1) et de son récepteur protéique (ZNHIT3 ou PIH1, respectivement) fusionnés respectivement aux extrémités N- et C-terminales de ladite protéine d'intérêt, et la protéine de fusion cyclisée résultante. Cela permet d'augmenter la thermostabilité et/ou d'aider à l'expression de protéines recombinantes et/ou d'aider à la purification de protéines recombinantes d'intérêt rendues plus solubles pour un meilleur rendement de production.

## Description

### Domaine technique de l'invention

L'invention concerne un nouveau procédé de cyclisation d'une protéine d'intérêt utilisant une étiquette protéique constituée d'un peptide spécifique (NUFIP1 ou TAH1) et de son récepteur protéique (ZNHIT3 ou PIH1, respectivement) fixés aux extrémités N- et C-terminales de ladite protéine d'intérêt, et la protéine de fusion cyclisée résultante. Cela permet d'augmenter la thermostabilité et/ou d'aider à l'expression de protéines recombinantes et/ou d'aider à la purification de protéines recombinantes d'intérêt rendues plus solubles pour un meilleur rendement de production.

Dans la description ci-dessous, les références entre crochets ([ ]) renvoient à la liste de références à la fin du texte.

### Etat de la technique

Depuis plusieurs décennies, les besoins industriels en protéines recombinantes sont croissants. Ces dernières sont généralement des biomolécules actives, produites par des micro-organismes hôtes (comme des bactéries ou des levures) génétiquement optimisés pour surproduire une protéine cible. C'est de cette façon que sont synthétisés, par exemple, l'insuline et ses dérivés, l'érythropoïétine (ou EPO), les enzymes lipolytiques (ou lipases) capables de digérer les graisses ou encore, plus récemment, les PET hydrolases qui possèdent la propriété de digérer le plastique. Les domaines d'application sont donc nombreux et porteurs. Ainsi la biocatalyse, phénomène rendu possible par les propriétés enzymatiques de certaines protéines recombinantes, est un procédé industriel éco-compatible, nombre des réactions enzymatiques se faisant dans l'eau et à des températures inférieures à 100°C. Se développent également de nouveaux outils diagnostiques et de recherche basés sur l'utilisation d'anticorps légers, encore appelés « nanobody » (ou nanocorps). Ces petites protéines, produites de façon recombinante, ont des capacités de fixation très forte à des ligands spécifiques et très variés, comme des toxines ou des protéines virales comme celles du SARS-COV2.

Même si l'essor de la biologie applicative a rendu possible la production à grande échelle de protéines recombinantes, il existe toutefois de nombreux écueils, de la découverte d'une cible intéressante à son exploitation intensive. Parmi eux, on peut citer le faible rendement de production, la faible solubilité ou encore l'agrégation de la protéine d'intérêt. Un point supplémentaire de vigilance est la stabilité de la protéine cible à la température, à l'agitation mécanique ou dans le temps. En effet, certaines enzymes, même si purifiées avec succès et utilisées à des températures modérées, se désactivent rapidement du fait de leur instabilité intrinsèque.

Les procédés de production d'une protéine recombinante étant couteux, des solutions améliorant les points mentionnés ci-dessus ont dues être envisagées pour faciliter les applications industrielles.

L'amélioration de la stabilité d'une protéine fait l'objet de nombreuses études dans la littérature. Une voie souvent empruntée est la mutagenèse. Cette dernière consiste à modifier, de façon aléatoire ou dirigée, la séquence en acides aminés de la protéine cible. Par exemple, il a été montré qu'un contenu riche en résidus chargés comme la lysine, l'arginine ou le glutamate était corrélé avec une plus grande résistance à la température (Kumar et al., 2000 ; Yokota et al., 2006 ; Strickler et al., 2006) [1-3]. Cependant cette approche peut altérer les capacités catalytiques ou de fixation de la protéine mutée. En effet, pour conserver les propriétés d'intérêt de la protéine native, il convient d'épargner les résidus de son site actif (ou dans son proche voisinage) ainsi que les résidus qui permettent de donner à la protéine sa forme tridimensionnelle.

Une autre méthode s'est montrée efficace pour augmenter la stabilité à la température et diminuer la propension à l'agrégation. Il s'agit de cycliser la protéine, en capturant ses extrémités. En effet, les régions aux extrémités des protéines sont des zones de forte entropie, propice à la désorganisation et donc pouvant déstabiliser le repliement tridimensionnel de la protéine, pourtant essentiel à sa fonction. Plusieurs méthodes de cyclisation sont possibles (Hayes et al., 2021) [4]. Une première approche repose sur des méthodes chimiques (activation ou réactivité intrinsèque d'acides aminés particuliers) qui aboutiront à la formation de liaisons covalentes entre extrémités. Une seconde approche consiste à utiliser une enzyme capable de prendre en charge des séquences spécifiques d'acides aminés implantées à chaque extrémité de la protéine, de les rapprocher dans l'espace et de former un lien covalent. C'est le mode de fonctionnement de la subtiligase, de la sortase ou encore de la butelase-1. Finalement, une troisième approche fait intervenir des étiquettes protéiques. C'est le cas des intéines, dont le mode d'action est à rapprocher de celui de l'épissage observé avec les acides nucléiques. Récemment ont également été développés des couples protéine/peptide qui, en plus d'une reconnaissance mutuelle spécifique, établissent entre eux, un pont isopeptidique covalent (Spy-ring, Snoop-ring, etc...) (Sun et al., 2019 ; Buldun et al., 2018 ; Wang et al., 2016) [5-7]. Cette propriété a été mise à profit pour clamper les extrémités de la phytase-C, enzyme utilisée par l'industrie agro-alimentaire pour libérer le phosphate de l'acide phytique et ainsi améliorer la digestibilité des aliments pour animaux. Cette cyclisation a permis d'augmenter la résistance thermique de l'enzyme et faciliter son utilisation dans un process industriel qui requiert une étape de stérilisation effectuée entre 75 et 90°C. Dans certains cas, comme celui de l'Ammonia-lyase, la cyclisation améliore également la stabilité thermique. Certaines PET hydrolases ont fait l'objet de cyclisation par des systèmes d'étiquettes protéiques, ce qui a permis d'augmenter leur activité catalytique et/ou leur stabilité (Hayes et al., 2023) [8].

### Description de l'invention

Dans ce contexte, les Inventeurs ont mis au point un système capable d'augmenter la surexpression et/ou la stabilité d'une protéine recombinante et/ou d'aider à sa purification en y intégrant des adaptateurs, ces derniers ayant la capacité de clamper les extrémités mobiles des protéines cibles. Ce procédé de cyclisation permet donc d'augmenter le rendement de production d'une protéine recombinante, par exemple d'une enzyme (e.g. PET hydrolase).

Le nouveau système proposé par les Inventeurs repose sur une étiquette protéique formée d'un couple protéine/peptide spécifique. Il s'agit d'un complexe très spécifique, thermorésistant et très affin entre un peptide et son récepteur protéique. Quand le peptide et son récepteur protéique sont respectivement fusionnés aux extrémités d'une protéine d'intérêt cible, ils interagissent pour former un clamp aboutissant à la cyclisation de la protéine d'intérêt. Cela permet d'augmenter l'expression et/ou la thermostabilité et/ou d'aider à la purification de la protéine d'intérêt rendue plus soluble pour un meilleur rendement de production.

Pour ce faire, les deux couples protéine/peptide ZNHIT3/NUFIP1 et PIH1/TAH1, qui ont déjà fait l'objet de nombreuses études fondamentales (Chagot et al., 2022 ; Henri et al., 2018 ; Quinternet et al., 2016 ; Quinternet et al., 2015 ; Quinternet et al., 2015 ; Rothé et al., 2014) [9-14], ont été sélectionnés et respectivement nommés ZN-ring et TP-ring. De façon intéressante, il a été montré que les complexes ZNHIT3/NUFIP1 d'*Homo sapiens,* de *Saccharomyces cerevisiae* et de *Plasmodium falciparum* adoptent des repliements tri-dimensionnels très similaires malgré un pourcentage d'identité faible entre les séquences protéiques de ces trois espèces (17 % au minimum) (Chagot et al., 2022 ; Quinternet et al.,2016) [9,12].

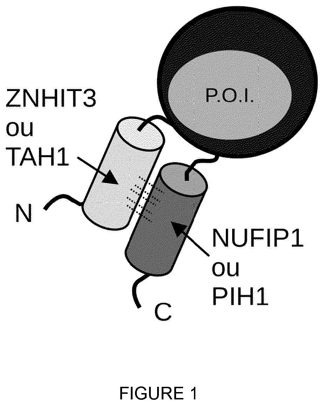

Le principe général du système de cyclisation selon l'invention est schématisé en Figure 1. ZNHIT3 et PIH1 (également nommés TRIP3 ou HIT1 et PID1D1, respectivement) sont tous les deux des domaines protéiques globulaires d'environ 80 résidus. NUFIP1 et TAH1 (également nommés RSA1 et RPAP3) sont des peptides linéaires d'environ 20 résidus ayant la particularité d'adopter un repliement tridimensionnel au contact de leur partenaire (récepteur protéique). Les complexes ZNHIT3/NUFIP1 et PIH1/TAH1 formés sont très stables et possèdent une température de dénaturation allant jusqu'à 90°C (Chagot et al., 2022) [9]. En deçà de cette température, l'association entre les deux partenaires demeure extrêmement forte du fait de la nature des interactions qui s'établit entre le peptide et son domaine protéique. En effet, de fortes interactions hydrophobes et électrostatiques ralentissent très fortement l'équilibre association/dissociation de ces complexes qui restent associés, même à très faible concentration. Contrairement aux systèmes Spy-ring ou Snoop-ring, ces complexes ne nécessitent pas la formation de pont isopeptidique pour être forts (Wang et al., 2016) [7]. La forte association à faible concentration est un avantage, la minimisation de la concentration en enzymes étant un paramètre pris en compte dans le calcul de rentabilité d'une application industrielle.

Un autre point avantageux des systèmes ZN-ring et TP-ring selon l'invention réside dans les composantes ZNHIT3 et PIH1 qui ne peuvent être solubles qu'en présence de leurs partenaires respectifs NUFIP1 et TAH1 (Quinternet et al., 2016 ; Rothé et al., 2014) [11,14]. Autrement dit, lorsque ZNHIT3 et PIH1 ne sont pas respectivement complexés à NUFIP1 et TAH1, ces domaines protéiques sont totalement insolubles. Quant à eux, et de façon intéressante, les complexes ZNHIT3/NUFIP1 et TAH1/PIH1 sont très solubles à haute concentration (Chagot et al., 2022 ; Henri et al., 2018 ; Quinternet et al., 2016 ; Quinternet et al., 2015 ; Quinternet et al., 2015 ; Rothé et al., 2014) [9-14]. Ainsi lorsque les systèmes TP-ring et ZN-ring sont utilisés comme étiquettes protéiques, un tri naturel des protéines cyclisées résultantes s'établit dès la première étape de purification. Cette étape consiste à séparer les protéines solubles des protéines insolubles. Ainsi, dans le cas d'une protéine d'intérêt intrinsèquement soluble fusionnée au système TP-ring et ZN-ring et pour laquelle le clamp ne s'est pas formé, les domaines ZNHIT3 et PIH non-complexés à NUFIP1 et TAH1 vont entrainer l'ensemble de la fusion protéique dans la fraction insoluble. Réciproquement, lorsque le clamp est formé et que la cyclisation s'opère, la fusion protéique sera entrainée dans la fraction soluble.

*A contrario,* les composantes protéine et peptide des systèmes Spy-ring et Snoop-ring sont solubles, indépendamment l'une de l'autre. Cela ne permet pas un tri drastique basé sur la solubilité des protéines cyclisées ou non.

L'utilisation des systèmes ZN-ring et TP-ring les rend d'autant plus attractifs qu'ils peuvent être facilement déclinés car les couples ZNHIT3/NUFIP1 et PIH1/TAH1 sont naturellement retrouvés dans différents organismes eucaryotes. Ici, pour le complexe ZNHIT3/NUFIP1, ce sont les séquences (protéiques) de *Plasmodium falciparum* qui ont été utilisées mais les Inventeurs ont déjà montré que les versions humaine et de levure de ce complexe résistent à des températures supérieures à 70°C (Chagot et al., 2022) [9]. Pour le complexe PIH1/TAH1, les séquences protéiques de *Saccharomyces cerevisiae* ont été utilisées (Quinternet et al., 2015) [12].

La présente invention a donc pour objet une protéine de fusion cyclisée comprenant un système de cyclisation constitué d'un peptide et de son récepteur protéique respectivement fusionnés aux extrémités N- et C-terminales d'une protéine d'intérêt, où ledit peptide est le peptide NUFIP1 ou TAH1 et ledit récepteur protéique est respectivement la protéine ZNHIT3 ou PIH1.

La présente invention a également pour objet l'utilisation d'une protéine de fusion cyclisée selon l'invention pour l'expression et/ou la purification d'une protéine d'intérêt.

La présente invention a également pour objet un procédé *in vitro* d'expression et de purification d'une protéine d'intérêt, caractérisé en ce qu'il comprend les étapes suivantes :
a) fusion d'un système de cyclisation constitué d'un peptide et de son récepteur protéique respectivement fusionnés aux extrémités N- et C-terminales d'une protéine d'intérêt ;
b) expression dans une cellule hôte de la protéine de fusion cyclisée obtenue à l'étape a) ;
c) purification de la protéine de fusion cyclisée ; et
d) optionnellement isolement de la protéine d'intérêt du système de cyclisation ; où ledit peptide est le peptide NUFIP1 ou TAH1 et ledit récepteur protéique est respectivement la protéine ZNHIT3 ou PIH1.

Selon un mode de réalisation particulier de la présente invention, le système de cyclisation ZNHIT3/NUFIP1 est celui de *P. falciparum, H. sapiens, S. cerevisiae* et/ou le système de cyclisation PIH1/TAH1 est celui de *S. cerevisiae.* En particulier le peptide NUFIP1 a la séquence SEQ ID NO : 4 ou une séquence ayant au moins 17 % d'identité avec SEQ ID NO : 4, le récepteur protéique ZNHIT3 a la séquence SEQ ID NO : 3 ou une séquence ayant au moins 18 % d'identité avec SEQ ID NO : 3, le peptide TAH1 a la séquence SEQ ID NO : 6, et/ou le récepteur protéique PIH1 a la séquence SEQ ID NO : 5.

Selon un mode de réalisation particulier de la présente invention, la cellule hôte est une cellule hôte eucaryote non humaine ou une cellule procaryote, par exemple choisie parmi *Saccharomyces cerevisiae, Escherichia coli* ou *Pichia pastoris.*

### Brève description des figures

Figure 1 représente le principe général du système de cyclisation selon l'invention. « P.O.I. » signifie « protéine d'intérêt »

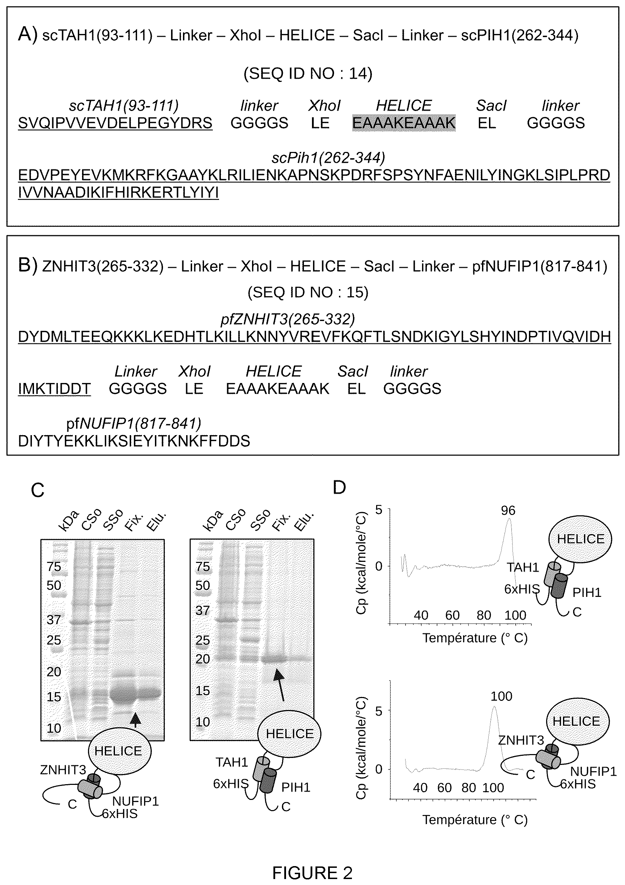

Figure 2 représente la séquence protéique d'une hélice cyclisée par le système TP-ring (A) et ZN-ring (B). Dans ce cas, des sites de clonage pour les enzymes de restrictions Xhol et Sacl ainsi que des régions flexibles (linker) poly-glycine (GGGGS) (SEQ ID NO : 1) ont été introduits de part et d'autre de la région à cycliser. Le système TP-ring est respectivement constitué des régions 93-111 et 262-344 des protéines TAH1 et PIH1 de *S. cerevisiae.* Le système ZN-ring est respectivement constitué des régions 265-332 et 817-841 des protéines ZNHIT3 et NUFIP1 de *P. falciparum. (C)* Suivi de purification des constructions indiquées en (A) et (B) insérées dans un plasmide pnEA et exprimées dans un souche E. coli BL21(DE3). Une étiquette 6xHIS est ajoutée en N-terminale des constructions. Cso, SSo, Fix, Elu et kDa se rapportent respectivement au culot de sonication (fraction insoluble), au surnageant de sonication (fraction soluble), à la fixation sur billes TALON, à l'éluat après addition d'imidazole sur les billes, et au marqueur de taille. (D) Courbes de température de demi-dénaturation pour l'hélice de séquence EAAAKEAAAK (SEQ ID NO : 2) cyclisée par les systèmes ZN-ring et TP-ring obtenues par calorimétrie différentielle à balayage.

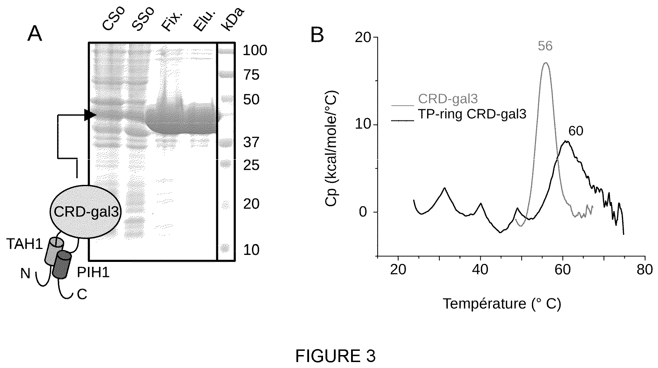

Figure 3 représente à titre d'exemple (A) le suivi de purification par gel de polyacrylamide dénaturant du CRD-gal3 inséré dans le système TP-ring et exprimé dans une souche *d'Escherichia coli* BL21(DE3). Cso, SSo, Fix, Elu et kDa se rapportent respectivement au culot de sonication (fraction insoluble), au surnageant de sonication (fraction soluble), à la fixation sur billes lactose-sépharose, à l'éluat après addition de D-lactose sur les billes, et au marqueur de taille. (B) Courbes de température de demi-dénaturation pour CRD-gal3 sauvage et inclus dans le système TP-ring, obtenues par calorimétrie différentielle à balayage.

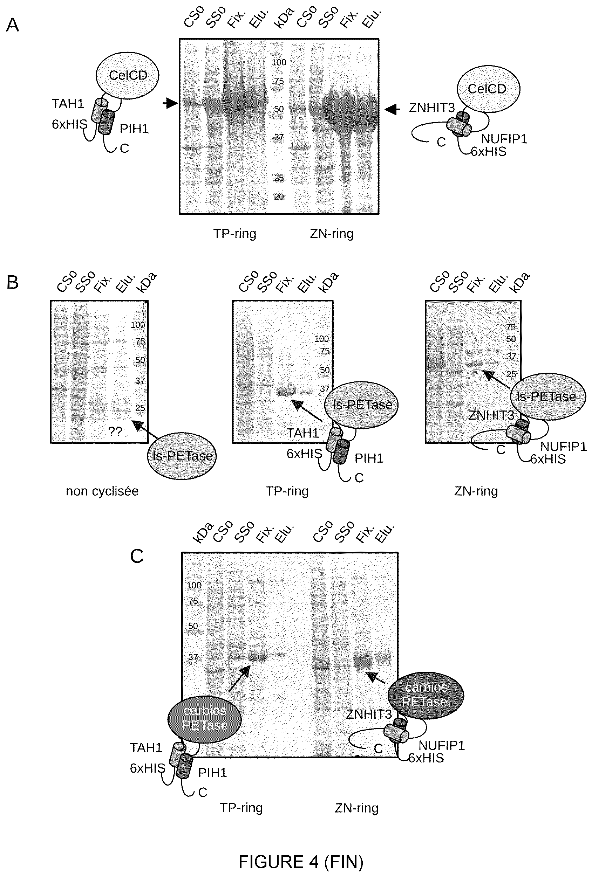

Figure 4 représente le suivi de purification par gel de polyacrylamide dénaturant, (A) de la cellulase Cel-CD, (B) de la PETase de *Ideonella sakaiensis* (Is-PETAse) et (C), de la PETase de Carbios (Carbios PETase), chacune insérée dans les systèmes TP-ring et ZN-ring. Is-PETase a été purifiée en version non-cyclisée (B). Les constructions ont été exprimées dans une souche d*'Escherichia coli* BL21(DE3). Cso, SSo, Fix, Elu et kDa se rapportent respectivement au culot de sonication (fraction insoluble), au surnageant de sonication (fraction soluble), à la fixation sur billes Lactose-sépharose ou TALON, à l'éluat après addition de D-Lactose ou d'imidazole sur les billes, et au marqueur de taille.

### EXEMPLES

### EXEMPLE 1 : PROCEDE DE CYCLISATION DE PROTEINES D'INTERET

Pour procéder à la cyclisation, un vecteur ADN plasmidique (exemple : pnEA, pET-20b ou pPICZ-A) est construit tel que la séquence d'une protéine ou d'un domaine protéique d'intérêt soit insérée entre les séquences ADN de ZNHIT3 et NUFIP1 ou de TAH1 et PIH1. Préférentiellement, ZNHIT3 ou TAH1 sont placés en amont de la protéine ou du domaine protéique d'intérêt, NUFIP1 et PIH1, respectivement en aval. Optionnellement, les séquences d'un court segment poly-glycine (de type GGGGS) (SEQ ID NO : 1) et/ou d'un site de restriction enzymatique de clonage peuvent être placées en aval et/ou en amont de la protéine ou du domaine protéique d'intérêt.

Les séquences des domaines de ZNHIT3 et NUFIP1 de *P. falciparum* et de TAH1 et PIH1 de *S*. *cerevisiae* respectivement utilisées dans les systèmes ZN-ring et TP-ring sont indiquées ci-dessous :
pfZNHIT3(265-332) : 
DYDMLTEEQKKKLKEDHTLKILLKNNYVREVFKQFTLSNDKIGYLSHYINDPTIVQVID
HIMKTIDDT (SEQ ID NO : 3)
pfNUFIP1(817-841) :
   DIYTYEKKLIKSIEYITKNKFFDDS (SEQ ID NO : 4)
scPIH1(262-344) : 
EDVPEYEVKMKRFKGAAYKLRILIENKAPNSKPDRFSPSYNFAENILYINGKLSIPLPR
DIVVNAADIKIFHIRKERTLYIYI (SEQ ID NO : 5)
scTAH1(93-111) :
   SVQIPVVEVDELPEGYDRS (SEQ ID NO : 6)

Les exemples de la cyclisation d'un court segment hélicoïdal de séquence EAAAKEAAAK (SEQ ID NO : 2) par les systèmes TP-ring et ZN-ring sont indiqués en Figures 2A et 2B. Les options segments poly-glycine et site de clonage sont figurées.

Le vecteur plasmidique à utiliser est adapté à l'organisme hôte choisi pour procéder à la cyclisation et peut embarquer des fonctions supplémentaires, comme des étiquettes protéiques assistant la purification (type histidine-tag ou 6xHIS). Dans un mode préférentiel, l'organisme hôte est une souche *Escherichia coli* BL21(DE3).

La cyclisation s'opère de façon spontanée lors de la production de la construction codée sur le plasmide par l'organisme hôte. Dans le cas de l'utilisation d'une souche *Escherichia coli* BL21(DE3), il convient de transformer les bactéries par le vecteur d'intérêt. Les bactéries sont ensuite cultivées dans un milieu permettant leur croissance. L'expression de la protéine ou du domaine protéique d'intérêt peut être induite par ajout d'IPTG (Isopropyl β-D-1-thiogalactopyranoside) en quantité suffisante dans le cas de l'utilisation d'un milieu de culture non auto-inductible. La purification de la protéine cyclisée peut être faite par des méthodes chromatographiques (affinité, exclusion de taille, etc.) à définir selon les propriétés biophysiques de la construction exprimée.

### EXEMPLE 2 : RESULTATS DE CYCLISATION DE PROTEINES D'INTERET

### I- Segment hélicoïdale de séquence EAAAKEAAAK (SEQ ID NO : 2)

Les séquences décrites en Figures 2A et 2B ont été introduites dans un vecteur pnEA qui code pour une étiquette 6xHIS supplémentaire en N-terminale de la construction. Ce vecteur a servi à transformer une souche *E. coli* BL21(DE3). Les bactéries ont ensuite été mises en culture sous agitation à 37°C dans un milieu LB. Lorsque l'absorbance du milieu (mesurée à 600 nm) a atteint une valeur de 0.7, la concentration en IPTG du milieu a été portée à 0.25 mM afin d'induire l'expression plasmidique de la protéine cyclisée. Après 16 heures à 20°C, un culot cellulaire a été récupéré par centrifugation puis dissous dans un tampon de lyse contenant 25 mM HEPES, pH 7.5, 300 mM NaCl et 10 mM Imidazole. Après centrifugation, la fraction soluble a été placée en contact d'une résine TALON à laquelle se fixe l'étiquette 6xHIS. Suite à trois lavages successifs avec du tampon de lyse, les protéines retenues sur la résine ont été éluées dans un tampon contenant 25 mM HEPES, pH 7.5, 300 mM NaCl et 300 mM Imidazole. L'analyse de la purification s'est faite sur gel de polyacrylamide en condition dénaturante et coloration par bleu de Coomassie.

La Figure 2C montre que l'hélice cyclisée avec les systèmes ZN-ring et TP-ring est présente dans la fraction soluble après lyse et centrifugation des bactéries (piste SSo). Une partie des construits se retrouve dans le culot de sonication (piste Cso), ce qui correspond aux construits pour lesquels la cyclisation ne s'est pas faite correctement. Ces derniers sont donc éliminés efficacement dès le début du processus de purification. Les construits cyclisés sont retenus efficacement sur la résine (piste Fix.). L'élution permet d'obtenir l'hélice EAAAKEAAAK (SEQ ID NO : 2) cyclisée en solution (piste Elu.)

La figure 2D montre par calorimétrie différentielle à balayage (DSC), que la température de demi-dénaturation des constructions est très élevée, soit 96 et 100°C pour l'hélice EAAAKEAAAK (SEQ ID NO : 2) respectivement cyclisée par les systèmes TP-ring et ZN-ring. Les mesures de DSC ont été effectuées avec des concentrations en protéines de 30 µM dans un tampon 25 mM HEPES, pH 7.5, 300 mM NaCl grâce à un instrument VP-DSC (MicroCal, Malvern, Ltd).

### II- Domaine CRD de la galectine-3 humaine (CRD-gal3)

Le domaine CRD-gal3 permet la purification de protéines (Demande internationale WO 2017/194888) [15]. Il s'agit d'un domaine capable de fixer le D-lactose. Ici, ce domaine a été cyclisé par le système TP-ring. Dans ce cas, les options segments poly-glycine et site de clonage ont été retenues. La séquence protéique de CRD-gal3 cyclisée a été introduite dans un vecteur pnEA.
TP-ring-CRD-gal3 :
**TAH1(93-111)**-Linker-*Xhol*-CRD-gal3(20-156)-*Sacl*-Linker-**PIH1(262-344)**

SVQIPVVEVDELPEGYDRSGGGGSLELIVPYNLPLPGGVVPKMLITILGTVKPNANRI
ALDFQRGNDVAFHFNPRFNENNRRVIVCNTKLDNNWGREERQSVFPFESGKPFKI
QVLVEPDHFKVAVNDAHLLQYNHRVKKLNEISKLGISGDIDLTSTSYTMIELGGGGSE

DVPEYEVKMKRFKGAAYKLRILIENKAPNSKPDRFSPSYNFAENILYINGKLSIPLP
RDIVVNAADIKIFHIRKERTLYIYI (SEQ ID NO: 7)

Le protocole de purification est identique à celui décrit pour l'hélice EAAAKEAAAK (SEQ ID NO: 2) cyclisée à la différence qu'une résine lactose-sépharose a été utilisée. Elle a permis de retenir CRD-gal3. Ici, l'élution s'est faite grâce au tampon de lyse supplémenté avec 200 mM D-lactose. La Figure 3A montre que CRD-gal3 cyclisé avec le système TP-ring est présent dans la fraction soluble après lyse et centrifugation des bactéries (piste SSo). Une partie des construits se retrouve dans le culot de sonication (piste Cso), ce qui correspond aux construits pour lesquels la cyclisation ne s'est pas faite correctement. Ces derniers sont donc éliminés efficacement dès le début du processus de purification. Les construits cyclisés sont retenus efficacement sur la résine (piste Fix.), démontrant ainsi l'innocuité du TP-ring sur la fonction et sur le repliement tri-dimensionnel de CRD-gal3. L'élution est également très efficace et permet d'obtenir CRD-gal3 cyclisé en solution (piste Elu.).

La figure 3B montre par calorimétrie différentielle à balayage (DSC), que la température de demi-dénaturation du domaine CRD-gal3 a été augmentée d'environ 7% lorsque cette protéine a été cyclisée par la méthode TP-ring (augmentation de 56 à 60°C). La cyclisation a donc permis d'augmenter la thermostabilité de CRD-gal3. Les mesures de DSC ont été effectuées avec des concentrations en protéines de 30 µM dans un tampon 25 mM HEPES, pH 7.5, 300 mM NaCl grâce à un instrument VP-DSC (MicroCal, Malvern, Ltd).

### III- Cellulase (Cel-CD)

Cette enzyme est capable de digérer la cellulose, et les produits de digestion possèdent un potentiel valorisable pour la conception de bio-carburant. Elle a été cyclisée par les systèmes TP-ring et ZN-ring. Dans ce cas, les options segments poly-glycine et sites de clonage ont été retenues. Les construits ci-dessous ont été introduits dans un vecteur plasmidique d'expression pnEA apportant une étiquette 6xHIS supplémentaire en N-terminal de Cel-CD cyclisée. Les séquences de Cel-CD cyclisée sont reportées ci-dessous :
TP-ring-Cel-CD :
   **TAH1(93-111**)-Linker-*Xhol*-Cel-CD(9-365)-*Sacl*-Linker-**PIH1(262-344)** 
SVQIPVVEVDELPEGYDRSGGGGSLEDNFKHLLGNDNVKRPSEAGALQLQEVDGQ
MTLVDQHGEKIQLRGMSTHGLQWFPEILNDNAYKALANDWESNMIRLAMYVGENG
YASNPELIKSRVIKGIDLAIENDMYVIVDWHVHAPGDPRDPVYAGAEDFFRDIAALYP
NNPHIIYELANEPSSNNNGGAGIPNNEEGWNAVKEYADPIVEMLRDSGNADDNIIIVG
SPNWSQRPDLAADNPIDDHHTMYTVHFYTGSHAASTESYPPETPNSERGNVMSNT
RYALENGVAVFATEWGTSQANGDGGPYFDEADVWIEFLNENNISWANWSLTNKNE
VSGAFTPFELGKSNATSLDPGPDQVWVPEELSLSGEYVRARIKGVNYELGGGGSE
DVPEYEVKMKRFKGAAYKLRILIENKAPNSKPDRFSPSYNFAENILYINGKLSIPLP
RDIVVNAADIKIFHIRKERTLYIYI (SEQ ID NO : 8)
ZN-ring-Cel-CD :
   **ZNHIT3(265-332)**-Linker-*Xhol*-Cel-CD(9-365)-*Sacl*-Linker-**NUFIP1(817-841)**

DYDMLTEEQKKKLKEDHTLKILLKNNYVREVFKQFTLSNDKIGYLSHYINDPTIVQVI
DHIMKTIDDTGGGGSLEDNFKHLLGNDNVKRPSEAGALQLQEVDGQMTLVDQHGE
KIQLRGMSTHGLQWFPEILNDNAYKALANDWESNMIRLAMYVGENGYASNPELIKS
RVIKGIDLAIENDMYVIVDWHVHAPGDPRDPVYAGAEDFFRDIAALYPNNPHIIYELA
NEPSSNNNGGAGIPNNEEGWNAVKEYADPIVEMLRDSGNADDNIIIVGSPNWSQRP
DLAADNPIDDHHTMYTVHFYTGSHAASTESYPPETPNSERGNVMSNTRYALENGV
AVFATEWGTSQANGDGGPYFDEADVWIEFLNENNISWANWSLTNKNEVSGAFTPF
ELGKSNATSLDPGPDQVWVPEELSLSGEYVRARIKGVNYELGGGGSDIYTYEKKLI
KSIEYITKNKFFDDS (SEQ ID NO: 9)

Le protocole de purification est identique à celui décrit pour CRD-gal3 à la différence qu'une résine TALON a été utilisée. Elle a permis de retenir l'étiquette 6xHIS introduite en N-terminale de Cel-CD cyclisée. L'élution s'est faite grâce au tampon de lyse supplémenté avec 300 mM Imidazole.

La Figure 4 montre que les construits recombinants de Cel-CD avec le système TP-ring ou ZN-ring (TP-ring ou ZN-ring, respectivement) sont également très largement surexprimés et majoritairement solubles. A nouveau, les construits non cyclisés sont efficacement éliminés dans la fraction insoluble (Cso).

### IV- PET hydrolases

Deux PET hydrolases ont été utilisées, l'une issue du microorganisme *Ideonella sakaiensis* (Is-PETase), l'autre faisant l'objet d'une exploitation industrielle par CARBIOS (carbios-PETase). Le protocole de purification mis en oeuvre est le même que celui utilisé pour Cel-CD cyclisée. Dans le cas de Is-PETase, l'option segment poly-glycine n'a pas été retenue. *A contrario,* cette option est incluse pour carbios-PETase. L'option site de clonage est présente dans les deux cas. Les séquences des protéines cyclisées sont reportées ci-dessous :
TP-ring-Is-PETase :
   **TAH1(93-111)** -*Xhol-*Is-PETase-*Sacl* **-PIH1(262-344)** 
SVQIPVVEVDELPEGYDRSLEMTNPYARGPNPTAASLEASAGPFTVRSFTVSRPSG
YGAGTVYYPTNAGGTVGAIAIVPGYTARQSSIKWWGPRLASHGFVVITIDTNSTLDQ
PSSRSSQQMAALRQVASLNGTSSSPIYGKVDTARMGVMGHSMGGGGSLISAANNP
SLKAAAPQAPWDSSTNFSSVTVPTLIFACENDSIAPVNSSALPIYDSMSRNAKQFLEI
NGGSHFCANSGNSNQALIGKKGVAWMKRFMDNDTRYSTFACENPNSTRVSDFRT
ANCSELEDVPEYEVKMKRFKGAAYKLRILIENKAPNSKPDRFSPSYNFAENILYING
KLSIPLPRDIVVNAADIKIFHIRKERTLYIYI (SEQ ID NO: 10)
ZN-ring-ls-PETase :
   **ZNHIT3(265-332)-** *Xhol*-Is-PETase- *Sacl***-NUFIP1(817-841)** 
DYDMLTEEQKKKLKEDHTLKILLKNNYVREVFKQFTLSNDKIGYLSHYINDPTIVQVI
DHIMKTIDDTLEMTNPYARGPNPTAASLEASAGPFTVRSFTVSRPSGYGAGTVYYP
TNAGGTVGAIAIVPGYTARQSSIKWWGPRLASHGFVVITIDTNSTLDQPSSRSSQQM
AALRQVASLNGTSSSPIYGKVDTARMGVMGHSMGGGGSLISAANNPSLKAAAPQA
PWDSSTNFSSVTVPTLIFACENDSIAPVNSSALPIYDSMSRNAKQFLEINGGSHFCA
NSGNSNQALIGKKGVAWMKRFMDNDTRYSTFACENPNSTRVSDFRTANCSELDIY
TYEKKLIKSIEYITKNKFFDDS (SEQ ID NO: 11)
TP-ring-carbios-PETase :
   **TAH1(93-111**)-Linker-*Xhol*-carbios-PETase-*Sacl*-Linker-**PIH1(262-344)** 
SVQIPVVEVDELPEGYDRSGGGGSLEMSNPYQRGPNPTRSALTADGPFSVATYTV
SRLSVSGFGGGVIYYPTGTSLTFGGIAMSPGYTADASSLAWLGRRLASHGFVVLVIN
TNSRFDGPDSRASQLSAALNYLRTSSPSAVRARLDANRLAVAGHSMGGGGTLRIAE
QNPSLKAAVPLTPWHTDKTFNTSVPVLIVGAEADTVAPVSQHAIPFYQNLPSTTPKV
YVELCNASHIAPNSNNAAISVYTISWMKLWVDNDTRYRQFLCNVNDPALCDFRTNN
 
RHCQELGGGGSEDVPEYEVKMRFKGAAYKLRILIENKAPNSKPDRFSPSYNFAE
NILYINGKLSIPLPRDIVVNAADIKIFHIRKERTLYIYI (SEQ ID NO: 12)
ZN-ring-carbios-PETase :
   **ZNHIT3(265-332)**-Linker-*Xhol*-carbios-PETase-*Sacl*-Linker**-NUFIP1(817-841)**

DYDMLTEEQKKKLKEDHTLKILLKNNYVREVFKQFTLSNDKIGYLSHYINDPTIVQVI
DHIMKTIDDTGGGGSLEMSNPYQRGPNPTRSALTADGPFSVATYTVSRLSVSGFG
GGVIYYPTGTSLTFGGIAMSPGYTADASSLAWLGRRLASHGFVVLVINTNSRFDGPD
SRASQLSAALNYLRTSSPSAVRARLDANRLAVAGHSMGGGGTLRIAEQNPSLKAAV
PLTPWHTDKTFNTSVPVLIVGAEADTVAPVSQHAIPFYQNLPSTTPKVYVELCNASHI
APNSNNAAISVYTISWMKLWVDNDTRYRQFLCNVNDPALCDFRTNNRHCQELGGG
GSDIYTYEKKLIKSIEYITKNKFFDDS (SEQ ID NO: 13)

La Figure 4B montre que la cyclisation par les systèmes TP-ring et ZN-ring a permis d'obtenir des quantités tout à fait significatives de Is-PETase soluble alors que cette dernière n'est pas identifiable lorsque produite non-cyclisée (visible en comparant l'intensité des bandes sur le gel de gauche avec celles observées sur les gels au milieu et à droite). Les Inventeurs comme la littérature (Fecker et al., 2018) [16], ont observé que cette enzyme à visée industrielle pouvait être totalement insoluble, nécessitant alors la mise en œuvre de protocoles complexes de renaturation. La cyclisation par les systèmes TP-ring et ZN-ring contourne cette mise en œuvre. Finalement, la Figure 4C montre que les systèmes de cyclisation selon l'invention permettent également de purifier une CARBIOS-PETase soluble dans de bonnes proportions.

### Liste de références

[1] Kumar et al., Protein Eng., 13(3) : 179-191, 2000
[2] Yokota et al., Science and Technology of Advanced Materials, 7(3): 255-262, 2006
[3] Strickler et al., Biochemistry, 45(9) : 2761-2766, 2006
[4] Hayes et al., Org. Biomol. Chem., 19(18) : 3983-4001, 2021
[5] Sun et al., N Biotechnol., 25 : 28-36, 2019
[6] Buldun et al., J. Am. Chem. Soc., 40(8) : 3008-3018, 2018
[7] Wang et al., Biotechnol. Biofuels, 31 ;9 :79. doi: 10.1186/s13068-016-0490-5, 2016
[8] Hayes et al., Scientific Reports, 13(1) : 1267. doi: 10.1038/s41598-023-27780-4, 2023
[9] Chagot et al., Biochemistry, 61(7) : 479-493, 2022
[10] Henri et al., Structure, 26(9) : 1196-1209.e8, 2018
[11] Quinternet et al., Structure, 24(10) : 1693-1706, 2016
[12] Quinternet et al., J. Mol. Biol., 427(17) : 2816-2839, 2015
[13] Quinternet et al., Biomol. NMR Assign., 2015
[14] Rothé et al., Nuc. Ac. Research, 2014
[15] Demande internationale WO 2017/194888
[16] Fecker et al., Biophys. J., 114(6) : 1302-1312, 2018

## Revendications

1. Protéine de fusion cyclisée comprenant un système de cyclisation constitué d'un peptide et de son récepteur protéique respectivement fusionnés aux extrémités N- et C-terminales d'une protéine d'intérêt, où ledit peptide est le peptide NUFIP1 ou TAH1 et ledit récepteur protéique est respectivement la protéine ZNHIT3 ou PIH1.

2. Protéine de fusion cyclisée selon la revendication 1, où le système de cyclisation ZNHIT3/NUFIP1 est celui de *P. falciparum, H. sapiens* ou *S. cerevisiae.*

3. Protéine de fusion cyclisée selon la revendication 1 ou 2, où le système de cyclisation PIH1/TAH1 est celui de *S. cerevisiae.*

4. Protéine de fusion selon l'une quelconque des revendications 1 à 3, où le peptide NUFIP1 a la séquence SEQ ID NO : 4 ou une séquence ayant au moins 17 % d'identité avec SEQ ID NO : 4, le récepteur protéique ZNHIT3 a la séquence SEQ ID NO : 3 ou une séquence ayant au moins 18 % d'identité avec SEQ ID NO : 3, le peptide TAH1 a la séquence SEQ ID NO : 6, et/ou le récepteur protéique PIH1 a la séquence SEQ ID NO : 5.

5. Utilisation d'une protéine de fusion cyclisée selon l'une quelconque des revendications 1 à 4 pour l'expression et/ou la purification d'une protéine d'intérêt.

6. Procédé *in vitro* d'expression et de purification d'une protéine d'intérêt, **caractérisé en ce qu'**il comprend les étapes suivantes :
a) fusion d'un système de cyclisation constitué d'un peptide et de son récepteur protéique respectivement fusionnés aux extrémités N- et C-terminales d'une protéine d'intérêt ;
b) expression dans une cellule hôte de la protéine de fusion cyclisée obtenue à l'étape a) ;
c) purification de la protéine de fusion cyclisée ; et
d) optionnellement isolement de la protéine d'intérêt du système de cyclisation ;
où ledit peptide est le peptide NUFIP1 ou TAH1 et ledit récepteur protéique est respectivement la protéine ZNHIT3 ou PIH1.

7. Procédé selon la revendication 6, où le système de cyclisation ZNHIT3/NUFIP1 est celui de *P. falciparum.*

8. Procédé selon la revendication 6 ou 7, où le système de cyclisation PIH1/TAH1 est celui de *S. cerevisiae.*

9. Procédé selon l'une quelconque des revendications 6 à 8, où la cellule hôte est une cellule eucaryote non humaine ou une cellule procaryote.

10. Procédé selon l'une quelconque des revendications 5 à 9, où le peptide NUFIP1 a la séquence SEQ ID NO : 4 ou une séquence ayant au moins 17 % d'identité avec SEQ ID NO : 4, le récepteur protéique ZNHIT3 a la séquence SEQ ID NO : 3 ou une séquence ayant au moin 18 % d'identité avec SEQ ID NO : 3, le peptide TAH1 a la séquence SEQ ID NO : 6, et/ou le récepteur protéique PIH1 a la séquence SEQ ID NO : 5.
